(19) Europäisches Patentamt
European Patent Office
Office européen des brevets

(11) Publication number: 0 160 246 B1

(12) EUROPEAN PATENT SPECIFICATION

(45) Date of publication of patent specification: 24.01.90

(51) Int. Cl.$^5$: C 07 D 249/20

(21) Application number: 85104559.1

(22) Date of filing: 15.04.85

# (54) A method for producing A 2-phenylbenzotriazole.

(30) Priority: 16.04.84 JP 77424/84

(43) Date of publication of application:
06.11.85 Bulletin 85/45

(45) Publication of the grant of the patent:
24.01.90 Bulletin 90/04

(84) Designated Contracting States:
CH DE FR GB IT LI NL

(56) References cited:
DE-A-2 003 860
US-A-3 262 943

PATENTS ABSTRACTS OF JAPAN, vol. 7, no. 11 (C-145)1156r, 18th January 1983; & JP - A - 57 167 976 (SHIPURO KASEI K.K.) 16-10-1982 &

CHEMICAL ABSTRACTS, vol. 98, no. 15, 11th April 1983, page 643, no. 126102b

(73) Proprietor: SUMITOMO CHEMICAL COMPANY, LIMITED
Kitahama 4-chome 5-33
Chuo-ku Osaka 541 (JP)

(72) Inventor: Kaneoya, Tatsuo
10-4-447, Sonehigashinocho-2-chome
Toyonaka-shi (JP)
Inventor: Maegawa, Yuzo
1, Kuwatacho-2-chome
Toyonaka-shi Osaka (JP)
Inventor: Okamura, Haruki
4, Shiginonishi-3-chome Joto-ku
Osaka (JP)

(74) Representative: Henkel, Feiler, Hänzel & Partner
Möhlstrasse 37
D-8000 München 80 (DE)

(56) References cited:
HOUBEN-WEYL: "METHODEN DER ORGANISCHEN CHEMIE", 4th edition, editor Eugen Müller, vol. X/3, Stickstoffverbindungen I, part 3, 1965, pages 430-431, Georg Thieme Verlag, Stuttgart, DE

EP 0 160 246 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a method for producing a 2-phenylbenzotriazole. More particularly, it relates to a method for producing a 2-phenylbenzotriazole represented by the general formula (III),

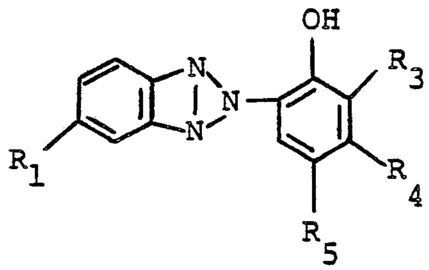

(III)

wherein $R_1$ represents a hydrogen or chlorine atom, $R_3$ and $R_5$ each represent a hydrogen atom, or a $C_1$—$C_{12}$ alkyl or a $C_1$—$C_8$ alkyl group substituted by phenyl group and $R_4$ represents a hydrogen atom, a hydroxy group or a methoxy group reducing a 2-phenylbenzotriazol-N-oxide represented by the general formula (II),

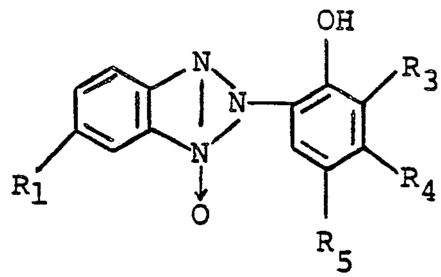

(II)

wherein $R_1$, $R_3$, $R_4$ and $R_5$ represent the same meanings as described above, in the presence of an alkali and a naphthoquinone optionally in the presence of a surface active agent wherein the reduction is carried out with one or more members selected from the group consisting of phosphorus, hypophosphorous acid and its salts and a method for producing a 2-phenylbenzotriazole represented byt he general formula (III),

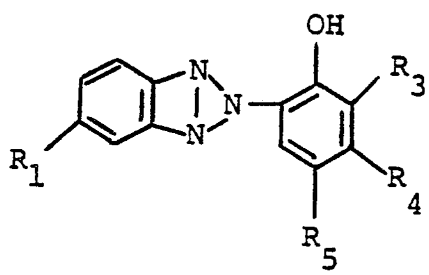

(III)

wherein $R_1$, $R_3$, $R_4$ and $R_5$ represent the same meanings as described above, which comprises a) reducing an o-nitroazobenzene represented by the general formula (I),

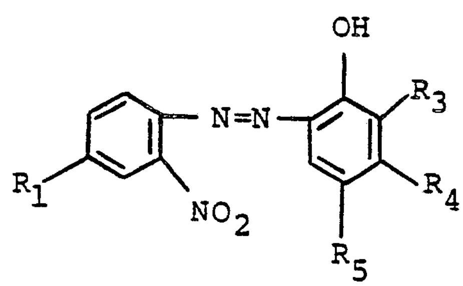

(I)

wherein $R_1$, $R_3$, $R_4$ and $R_5$ represent the same meanings as described above, to obtain a 2-phenylbenzotriazol-N-oxide represented by the general formula (II),

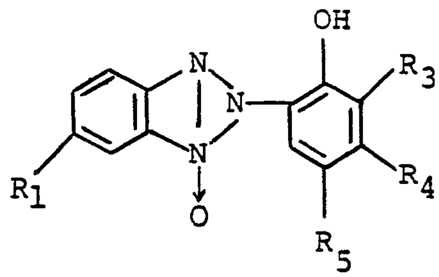

(II)

wherein $R_1$, $R_3$, $R_4$ and $R_5$ represent the same meanings as described above,
and b) reducing the resulting compound (II) both steps a) and b) being carried out in the presence of an alkali and a naphthoquinone and optionally in the presence of a surface active agent, wherein both reductions are carried out with one or more members selected from the group consisting of phosphorus, hypophosphorous acid and its salts.

A 2-phenylbenzotriazole, the object of the present invention, represented by the general formula (III) is useful as an ultraviolet absorber, and an object of the present invention is to provide a method for producing said triazole at low costs and in an industrially very advantageous manner.

The conventionally well-known general method for synthesizing a 2-phenylbenzotriazole represented by the general formula (III) is reduction of the corresponding o-nitroazobenzene [general formula (I)] or 2-phenylbenzotriazol-N-oxide [general formula (II)] with a reducing agent or by electrolytic reduction. But, these well-known methods have their defects, being not a quite satisfactory method in industrial scales. For example, Japanese Patent Publication No. 5934/1962 discloses a method wherein o-nitroazobenzenes are reduced into the corresponding 2-phenylbenzotriazoles with zinc powder in an aqueous alkali solution, but this method which uses zinc has a problem such that: The reaction rate is so slow that reaction at high temperature needs to be continued for a long time, and besides that there easily occur side reactions such as reductive cleavage of the azo group, and dehalogenation when the desired product is a halogen atom-containing compound, and the like. It was therefore difficult to obtain the desired product of high purity in high yields. In addition, this method which uses zinc causes environmental problems such as generation of zinc-containing sludge and waste water, so that it was a method having many industrial problems.

Also, the above patent publication suggests reduction with alkali sulfide. But, the present inventors' actual investigation showed that by-products were produced in large quantities to fail to obtain good results in not only reaction yield but also quality. This reduction method with alkali sulfide is therefore not practical.

Also, Chemical Abstracts, Vol. 24, pp. 2060 (published in 1930) reports electrolytic reduction of o-nitroazobenzenes with copper electrodes in an aqueous dilute sodium hydroxide solution. This method, however, has problems that not only a special apparatus of electrolytic plants is required, but also the yield is low (50 to 70%), so that it is not a practical method.

A method for synthesizing 2-phenylbenzotriazoles by hydrogenation of o-nitroazobenzenes is disclosed in Japanese Patent Application Kokai (Laid-Open) Nos. 138676/1976, 138677/1976, 138678/1976 and 138679/1976. This method, however, not only requires a special apparatus of autoclaves, but also gives as low a yield as about 80% at best, so that they are not a satisfactory method.

Japanese Patent Application Kokai (Laid-Open) No. 88072/1975 discloses a reduction method with hydrazine. But, the present inventors' actual investigation showed that it was difficult to obtain the desired product in high purity probably because side reactions easily occur during the reaction.

Also, the well-known methods described above had a serious problem as follows in addition to the foregoing problems: When water is contained in the reaction system, the desired reaction, particularly reduction of N-oxides of the general formula (II) into triazoles of the general formula (III) is markedly inhibited to cause an extreme increase in the amount of impurities. Because of this, the well-known methods employ complicated techniques which are industrially undesirable as follows: o-Nitroazobenzenes of the general formula (I) commonly available as a wet cake is dried expressly before use; and when the o-nitroazobenzene wet cake is used as such, the reaction is first allowed to proceed to the stage of the corresponding N-oxide, the N-oxide is once isolated and dried or water in the reaction system is removed by azeotropic dehydration or the like, and then the subsequent reaction is advanced. This was one of the problems inherent to the well-known methods.

Japanese Patent Application Kokai (Laid-Open) No. 167976/1982 discloses a method for producing a 2-phenylbenzotriazole by using formaldehyde as a reducing agent. This method, however, is still unsatisfactory in respect to the reaction yield and product purity. Further, according to this method, acetic acid is by-produced by the oxidation of formaldehyde, and also other by-products are produced, resulting high load of the waste water to the environment. Therefore, it has some problems particularly in an industrial scale operation.

The present inventors extensively studied to solve the defects of these conventionally well-known methods, and as a result, found a very advantageous method which provides the desired product in high yield and in high purity and besides is easy to carry out industrially. The present inventors thus completed

the present invention.

According to the present invention, there is provided a method for producing a 2-phenylbenzotriazole represented by the general formula (III),

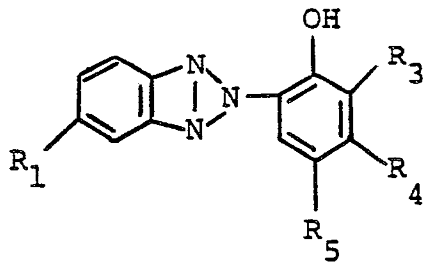

(III)

wherein $R_1$, $R_3$, $R_4$ and $R_5$ represent the same meanings as described above, which comprises two steps:

(1) First step for obtaining a 2-phenylbenzotriazol-N-oxide represented by the general formula (II),

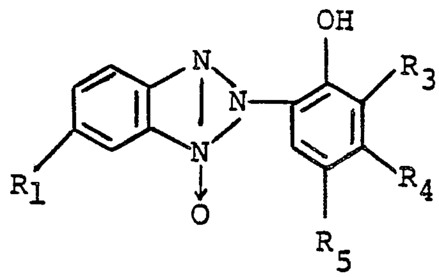

(II)

wherein $R_1$, $R_3$, $R_4$ and $R_5$ represent the same meanings as described above, by reducing an o-nitroazobenzene represented by the general formula (I),

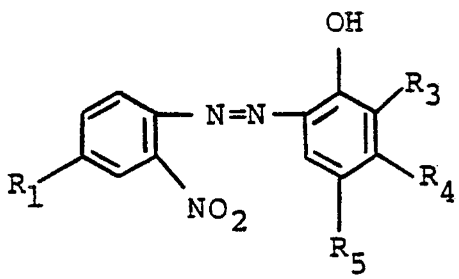

(I)

wherein $R_1$, $R_3$, $R_4$ and $R_5$ represent the same meanings as described above, with one or more members selected from the group consisting of phosphorus, hypophosphorous acid and its salts in the presence of an alkali and a naphthoquinone, and

(2) Second step for reducing the resulting 2-phenylbenzotriazol-N-oxide of the general formula (II), which may or may not be separated from the reaction system after completion of the first step, with one or more members selected from the group consisting of phosphorus, hypophosphorous acid and its salts in the presence of an alkali and a naphthoquinone. The present invention will be illustrated in detail hereinafter.

An o-nitroazobenzene of the general formula (I), a starting material of the present invention, can easily be produced by diazotizing an o-nitroaniline, generally, in an aqueous solvent to obtain the diazo compound which is then coupled with a phenol. The resulting o-nitroaniline is obtained as a wet cake by filtration, etc. In the present invention, the wet cake can be used as such if its water content is 45% or less, but it may be dried before use.

Specific examples of o-nitroazobenzenes include for example the following:

2-Nitro-4-chloro-2'-hydroxy-3'-tert-butyl-5'-methylazobenzene
2-Nitro-2'-hydroxy-5'-methylazobenzene
2-Nitro-2'-hydroxy-5'-tert-octylazobenzene
2-Nitro-4-chloro-2'-hydroxy-3',5'-di-tert-butylazobenzene
2-Nitro-2'-hydroxy-3',5'-di-tert-amylazobenzene
2-Nitro-2'-hydroxy-3',5'-di-tert-butylazobenzene
2-Nitro-2'-hydroxy-3'-tert-butyl-5'-methylazobenzene

2-Nitro-2',4'-dihydroxyazobenzene
2-Nitro-4-chloro-2',4'-dihydroxyazobenzene
2-Nitro-2'-hydroxy-4'-methoxyazobenzene
2-Nitro-4-chloro-2'-hydroxy-3',5'-di-tert-amylazobenzene
2-Nitro-2'-hydroxy-5'-tert-amylazobenzene
2-Nitro-4-chloro-2'-hydroxy-5'-tert-amylazobenzene
2-Nitro-2'-hydroxy-3',5'-di(α,α-dimethylbenzyl)azobenzene
2-Nitro-4-chloro-2'-hydroxy-3',5'-di-(α,α-dimethylbenzyl)azobenzene
2-Nitro-2'-hydroxy-3'-α-methylbenzyl-5'-methylazobenzene
2-Nitro-4-chloro-2'-hydroxy-3'-α-methylbenzyl-5'-methylazobenzene
2-Nitro-2'-hydroxy-5'-n-dodecylazobenzene
2-Nitro-4-chloro-2'-hydroxy-5'-n-dodecylazobenzene
2-Nitro-2'-hydroxy-3',5'-di-tert-octylazobenzene
2-Nitro-4-chloro-2'-hydroxy-3',5'-di-tert-octylazobenzene
2-Nitro-4-chloro-2'-hydroxy-5'-tert-octylazobenzene

A 2-phenylbenzotriazol-N-oxide represented by the general formula (II) is the corresponding N-oxide of the compound of the general formula (I) given above.

The reaction of the present invention can be carried out e.g. in water and/or in an organic solvent. When an organic solvent is used, it is for example an alcohol (e.g. methanol, ethanol, isopropanol, butanol), a cellosolve solvent (e.g. ethylene glycol monomethyl ether, ethylene glycol monoethyl ether), a polar solvent (e.g. dimethylformamide, dimethyl sulfoxide, alkylsulfone, acetone, acetonitrile), an aromatic hydrocarbon (e.g. benzene, toluene, xylene), an aliphatic hydrocarbon (e.g. n-hexane, n-heptane, ligroin) and the like. A preferred amount of the solvent used is 3 to 8 times by weight based on an o-nitroazobenzene of the general formula (I) or a 2-phenylbenzotriazol-N-oxide of the general formula (II). Of course, amounts more than that can also be used without hindrance to the reaction.

A surface active agent may be used in the present invention, and its amount is not particularly limited. Generally, the amount is 0 to 10 wt.%, preferably 0 to 5 wt.% based on an o-nitroazobenzene of the general formula (I) or a 2-phenylbenzotriazol-N-oxide of the general formula (II). The kind of the surface active agent is not particularly limited, and for example, the following are given: Turkey red oil, alkylarylsulfonates, higher alcohol sulfuric acid esters, polyoxyethylene alkyl ether, polyoxyethylene phenyl ether, polyoxyethylene sulfate, polyoxyethylene sorbitan fatty acid esters, polyoxyethylene acyl esters and polyhydric alcohols such as ethylene glycol, propylene glycol, butylene glycol, hexylene glycol, polyethylene glycol, polypropylene glycol, glycerin, pentaglycerol, etc.

As the alkali used in the present invention, the hydroxide, carbonate, hydrogencarbonate, etc. of alkali metals or alkaline earth metals are used. For example, even commonly used alkalis such as sodium hydroxide, potassium hydroxide, etc. may be used. The amount of the alkali used is preferably 2 to 6 moles based on 1 mole of an o-nitroazobenzene of the general formula (I) or a 2-phenylbenzotriazol-N-oxide of the general formula (II). Amounts lower than this tend to lower the reaction rate.

As a naphthoquinone used in the present invention, there are given for example 1,2-naphthoquinone, 1,4-naphthoquinone, 2,6-naphthoquinone, 2,3-dichloro-1,4-naphthoquinone and naphthoquinones substituted with an alkyl group at the nucleus. The amount of a naphthoquinone used at the step to obtain a 2-phenylbenzotriazol-N-oxide of the general formula (II) (firt step) is 0.002 to 0.08 mole, preferably 0.008 to 0.06 mole based on 1 mole of an o-nitroazobenzene of the general formula (I). Said amount used at the step to obtain the desired product, a 2-phenylbenzotriazole, (second step) is 0.01 to 0.1 mole, preferably 0.02 to 0.08 mole based on 1 mole of the N-oxide of the general formula (II), and it is added additionally to the reaction system when the reaction is continued without isolating said N-oxide after the first step.

In the present invention, phosphorus or hypophosphorous acid is used as a reducing agent. The form of phosphorus used includes for example liquid phosphorus, yellow phosphorus, red phosphorus, brown phosphorus, black phosphorus, etc. Of these, yellow phosphorus and red phosphorus are preferred. The amount of phosphorus used at the step to obtain a 2-phenylbenzotriazol-N-oxide of the general formula (II) (first step) is generally 0.2 to 2 moles based on 1 mole of an o-nitroazobenzene of the general formula (I). Said amount used at the step to obtain the desired product, a 2-phenylbenzotriazole of the general formula (III), (second step) is generally 0.2 to 2 moles based on 1 mole of the N-oxide of the general formula (II), and it is added additionally to the reaction system when the reaction is continued without isolating said N-oxide after the first step.

When hypophosphorous acid is used, the form of the acid includes for example a free acid and the salts of the acid with an alkali metal or alkaline earth metal such as hypophosphorous acid, sodium hypophosphite, potassium hypophosphite, etc. The amount of the acid used at the step to obtain a 2-phenylbenzotriazol-N-oxide of the general formula (II) (first step) is generally 0.5 to 4 moles based on 1 mole of an azobenzene of the general formula (I). Said amount used at the step to obtain the desired product, a 2-phenylbenzotriazole of the general formula (III), (second step) is 0.5 to 4 moles based on 1 mole of the N-oxide of the general formula (II), and it is added additionally to the reaction system when the reaction is continued without isolating said N-oxide after the first step.

The reaction temperature used in the present invention is generally selected from a relatively mild temperature range of 20° to 50°C at the first step to obtain an N-oxide of the general formula (II). While said

temperature is generally 60° to 100°C at the second step to obtain the desired product of the present invention, a 2-phenylbenzotriazole.

As described above, the method of the present invention comprises the first and second steps. The reaction of the second step may be carried out after isolating an N-oxide of the general formula (II) which is the product of the first step, or without isolating the N-oxide. In the latter case, said reaction is carried out after additionally adding the phosphorus compound and naphthoquinone.

Next, the present invention will be illustrated with reference to the following examples, but it is not limited to these examples only.

Example 1

19 Grams of 2-nitro-4-chloro-2'-hydroxy-3'-tert-butyl-5'-methylazobenzene and 7.4 g of sodium hydroxide were added to 100 g of a 95% aqueous isopropyl alcohol solution, and after raising the temperature to 60° to 65°C, 0.3 g of polyoxyethylene lauryl ether (trade name, Emulgen 106; produced by Kao Atlas Co.). After gradually cooling the reaction mixture to 30°C, 0.4 g of 2,3-dichloro-1,4-naphthoquinone was added and then 0.6 g of yellow phosphorus was further added in portions over about 1 hour, while properly cooling the reaction system in a water bath since addition of phosphorus was accompanied by generation of heat. The reaction system was stirred at 30° to 35°C for about 1 hour. After confirming complete disappearance of the red color of the azobenzene, the reaction system was neutralized to a pH of 7 to 8 with aqueous sulfuric acid. The deposited yellow crystal was separated by filtration, washed with 200 ml of water and then with a little isopropyl alcohol and dried to obtain 17.6 g of 2-(2-hydroxy-3-tert-butyl-5-methylphenyl)-5-chlorobenzotriazol-N-oxide. Yield, 97%. Melting point, 160°—163°C.

Example 2

Procedure was carried out in the same manner as in Example 1 except that 21.7 g of a 10% aqueous sodium hypophosphite solution was used in place of yellow phosphorus, to obtain 17.5 g of 2-(2-hydroxy-3-tert-butyl-5-methylphenyl)-5-chlorobenzotriazol-N-oxide. Yield, 96.4%. Melting point, 161°—163°C.

Example 3

A mixture comprising 17.6 g of 2-(2-hydroxy-3-tert-butyl-5-methylphenyl)-5-chlorobenzotriazol-N-oxide, 100 g of isopropyl alcohol, 5.2 g of sodium hydroxide and 0.3 g of ethylene glycol was adjusted to a temperature of 30° to 35°C. After adding 0.5 g of 2,3-dichloro-1,4- naphthoquinone to this mixture, 0.6 g of yellow phosphorus was further added thereto over 30 minutes, and then the temperature was raised to 75° to 80°C over about 1 hour. On continuing reaction with stirring at 75° to 80°C for 2 hours, the N-oxide disappeared and reduction reached completion. Thereafter, 100 ml of water was added to the reaction mixture, and the mixture was neutralized to a pH of 7 to 8 with sulfuric acid. The deposited yellowish white crystal was separated by filtration, washed with water and then with a little isopropyl alcohol and dried to obtain 15.9 g of 2-(2-hydroxy-3-tert-butyl-5-methylphenyl)-5-chlorobenzotriazole. Yield, 94.9%. Melting point, 138°—140°C.

Example 4

Procedure was carried out in the same manner as in Example 3 except that 4.2 g of sodium hypophosphite was used in place of yellow phosphorus, to obtain 16.0 g of 2-(2-hydroxy-3-tert-butyl-5-methylphenyl)-5-chlorobenzotriazole. Yield, 95.5%. Melting point, 138°—140°C.

Example 5

50 Grams of 2-nitro-2'-hydroxy-5'-methylazobenzene was dissolved by heating in a solution comprising 200 ml of water and 29 g of sodium hydroxide. To the resulting solution were added 1 g of polyoxyethylene lauryl ether (trade name, Emulgen 106; produced by Kao Atlas Co.) and 2 g of 2,3-dichloro-1,4-naphthoquinone, and then 0.6 g of yellow phosphorus was further added at 30° to 35°C over 30 minutes. On continuing reaction with stirring at 30° to 35°C for further 1 hour, the red color of the azobenzene disappeared and reduction reached completion. The reaction mixture was neutralized to a pH of 7 to 8 with sulfuric acid, and the deposited yellow crystal was separated by filtration, thoroughly washed with water and dried to obtain 46 g of 2-(2-hydroxy-5-methylphenyl)benzotriazol-N-oxide. Yield, 98.1%. Melting point, 138°—140°C.

Example 6

Procedure was carried out in the same manner as in Example 4 except that 46 g of 2-(2-hydroxy-5-methylphenyl)benzotriazol-N-oxide was used in place of 2-(2-hydroxy-3-tert-butyl-5-methylphenyl)-5-chlorobenzotriazol-N-oxide, to obtain 41 g of 2-(2-hydroxy-5-methylphenyl)benzotriazole as a pale yellow crystal. Yield, 95.5%. Melting point, 128°—130°C.

Comparative Example 1

Procedure was carried out in the same manner as in Example 1 except that 9.9 g of a 31% aqueous sodium hydrosulfide solution was used in place of yellow phosphorus, to obtain 17 g of 2-(2-hydroxy-3-tert-butyl-5-methylphenyl)-5-chlorobenzotriazol-N-oxide. Purity, 88.5%. Yield, 83.2%.

Comparative Example 2

Procedure was carried out in the same manner as in Example 3 except that 9.9 g of a 31% aqueous sodium hydrosulfide solution was used in place of yellow phosphorus, to obtain 16.3 g of 2-(2-hydroxy-3-tert-butyl-5-methylphenyl)-5-chlorobenzotriazole. Purity, 79.5%. Yield, 77.5%. The crystal obtained was colored reddish brown, and the color could not be decolored even by purification by recrystallization.

Example 7

19 Grams of 2-nitro-4-chloro-2'-hydroxy-3'-tert-butyl-5'-methylazobenzene and 7.8 g of sodium hydroxide were added to 100 g of isopropyl alcohol, and the temperature was raised to 60°C with stirring. After adding 0.9 g of ethylene glycol, the reaction mixture was gradually cooled to 30°C, and 0.6 g of 2,3-dichloro-1,4-naphthoquinone was added. Thereafter, 0.6 g of red phosphorus was further added in portions over about 1 hour, while properly cooling the reaction mixture in a water bath since addition of phosphorus was accompanied by generation of heat. On carrying out stirring at 30° to 35°C for more than 1 hour, the azobenzene disappeared, and 2-(2-hydroxy-3-tert-butyl-5-methylphenyl)-5-chlorobenzotriazole-N-oxide was formed. In order to complete the reaction, 0.3 g of ethylene glycol, 5.5 g of sodium hydroxide and 0.6 g of 2,3-dichloro-1,4-naphthoquinone were added, 0.6 g of red phosphorus was then added at 30° to 35°C over 30 minutes, and the temperature was raised to 75°C over about 1 hour. Subsequently, on continuing stirring at 75° to 80°C for 2 hours, the N-oxide disappeared, and reduction reached completion. To the reaction mixture was added 100 ml of water, and the mixture was neutralized to a pH of 7 to 8 with sulfuric acid. The deposited yellowish white crystal was separated by filtration, washed with 200 ml of water and then with a little isopropyl alcohol and dried to obtain 16.3 of 2-(2-hydroxy-3-tert-butyl-5-methylphenyl)-5-chlorobenzotriazole. Yield, 94.5%. Purity, 99.9%. Melting point, 137°—140°C.

Comparative Example 3

Procedure was carried out in the same manner as in Example 7 except that 9.9 g of a 31% aqueous sodium hydrosulfide solution was used in place of red phosphorus, to obtain 14 g of 2-(2-hydroxy-3-tert-butyl-5-methylphenyl)-5-chlorobenzotriazole. Purity, 78.5%. Yield, 64.5%. The crystal obtained was colored reddish brown, and the color could not be decolored even by purification by recrystallization, etc.

Example 8

Procedure was carried out in the same manner as in Example 7 except that 2.7 g of hypophosphorous acid liquefied by heating to 30° to 40°C was used in place of red phosphorus, to obtain 16.3 g of 2-(2-hydroxy-3-tert-butyl-5-methylphenyl)-5-chlorobenzotriazole as a yellowish white crystal. Yield, 94.5%. Melting point, 137°—140°C.

Example 9

Procedure was carried out in the same manner as in Example 7 except that polyoxyethylene lauryl ether (trade name, Emulgen 106; a nonionic surface active agent Produced by Kao Atlas Co.) was used in place of ethylene glycol, to obtain 16.1 g of 2-(2-hydroxy-3-butyl-5-methylphenyl)-5-chlorobenzotriazole. Yield, 93.5%. Melting point, 137°—140°C.

Example 10

Procedure was carried out in the same manner as in Example 7 except that ethylene glycol was not used, to obtain 15.9 g of 2-(2-hydroxy-3-tert-butyl-5-methylphenyl)-5-chlorobenzotriazole. Yield, 92%. Melting point, 137°—141°C.

Example 11

A mixture comprising 50 g of 2-nitro-4-chloro-2'-hydroxy-3',5'-di-tert-butylazobenzene, 205 g of ethylene glycol monoethyl ether, 18 g of sodium hydroxide and 2.5 g of ethylene glycol was stirred for 30 minutes, 1.5 g of 2,3-dichloro-1,4-naphthoquinone was then added and the temperature of the resulting mixture was adjusted to 30° to 35°C. Thereafter, 1.4 g of red phosphorus was added at 30° to 35°C over 30 minutes, while properly cooling the reaction mixture in a water bath since addition of phosphorus was accompanied by generation of heat. After completion of the addition, reaction was carried out at 35° to 40°C for 1 hour. After confirming disappearance of the azobenzene, 0.6 g of ethylene glycol, 12.3 g of sodium hydroxide and 1.5 g of 2,3-dichloro-1,4-naphthoquinone were added, 1.4 g of red phosphorus was then further added over 30 minutes, and subsequently the temperature was gradually raised to 85°C over 1 hour. On carrying out reaction at 85° to 90°C for 2 hours, reduction reached completion. Thereafter, 250 ml of water was added to the reaction mixture, and the mixture was neutralized to a pH of 7 to 8 with sulfuric acid. The deposited yellow crystal was separated by filtration, washed with 400 ml of water and dried to obtain 42.2 g of 2-(2-hydroxy-3,5-di-tert-butylphenyl)-5-chlorobenzotriazole. Yield, 92%. Melting point, 153°—155°C.

Example 12

Procedure was carried out in the same manner as in Example 11 except that 10.2 g of sodium hypophosphite was used in place of red phosphorus, to obtain 42 g of 2-(2-hydroxy-3,5-di-tert-butylphenyl)-5-chlorobenzotriazole. Yield, 91.6%. Melting point, 153°—155°C.

Comparative Example 4

19 Grams of 2-nitro-4-chloro-2′-hydroxy-3′-tert-butyl-5′-methylazobenzene and 8.7 g of sodium hydroxide were added to 100 g of methyl cellosolve, and after stirring at room temperature for 30 minutes, 13.1 g of zinc powder was added. After addition, stirring was continued for 1 hour, the temperature was raised to 60°C, and reaction was carried out for further 5 hours with stirring. After confirming disappearance of the raw material and the intermediate (N-oxide), 300 g of toluene was added to the reaction mixture, followed by filtration. The toluene layer was washed with aqueous hydrochloric acid, and toluene was removed by evaporation to obtain 14.8 g of 2-(2-hydroxy-3-tert-butyl-5-methylphenyl)-5-chlorobenzotriazole. Purity, 88%. Yield, 75.5%.

Comparative Example 5

27.8 Grams of 2-nitro-4-chloro-2′-hydroxy-3′-tert-butyl-5′-methylazobenzene and 0.9 g of potassium hydroxide were added to 60 g of diethylene glycol dimethyl ether, and the resulting mixture was heated to 70°C with stirring. Thereafter, 2.6 g of 80% hydrazine hydrate was added dropwise at 70°C over 1 hour, and the temperature was raised to 90°C, followed by stirring at the same temperature for 2 hours. Thereafter, the temperature was further raised to 135°C, and 2.6 g of 80% hydrazine hydrate was added dropwise at the same temperature over 2 hours while distilling water out of the reaction system. Stirring was continued at 135°C for further 2 hours, and after confirming disappearance of the raw material and the intermediate product (N-oxide), the reaction mixture was cooled to 90°C. Subsequently, 44 g of 2.7% hydrochloric acid was added, and the reaction mixture was discharged into 270 ml of 80°C water. The deposited yellowish brown crystal was separated by filtration, washed with water and dried to obtain 25.5 g of 2-(2-hydroxy-3-tert-butyl-5-methylphenyl)-5-chlorobenzotriazole. Purity, 69%. Yield, 69.6%.

Example 13

Procedure was carried out in the same manner as in Example 7 except that 25.3 g of a 2-nitro-4-chloro-2′-hydroxy-3′-tert-butyl-5′-methylazobenzene wet cake containing 25 wt.% of water was used in place of the dried product of the compound, to obtain 16 g of 2-(2-hydroxy-3-tert-butyl-5-methylphenyl)-5-chlorobenzotriazole. Yield, 92.7%. Melting point, 137°—141°C.

Comparative Example 6

Procedure was carried out in the same manner as in Example 13 except that 3.6 g of 90% paraformaldehyde was used in place of red phosphorus, to obtain 14.4 g of 2-(2-hydroxy-3-tert-butyl-5-methylphenyl)-5-chlorobenzotriazole. Purity, 94%. Yield, 78.5%.

**Claims**

1. A method for producing a 2-phenylbenzotriazole represented by the general formula (III),

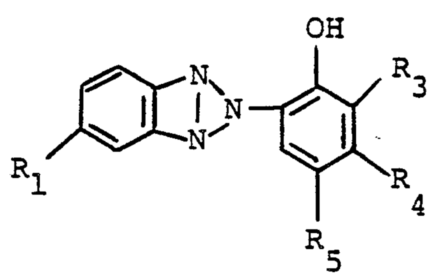

(III)

wherein $R_1$ represents a hydrogen or chlorine atom, $R_3$ and $R_5$ each represent a hydrogen atom, a $C_1$—$C_{12}$ alkyl group or a $C_1$—$C_8$ alkyl group substituted by phenyl group, and $R_4$ represents a hydrogen atom, a hydroxy group or a methoxy group, by reducing a 2-phenylbenzotriazol-N-oxide represented by the general formula (II),

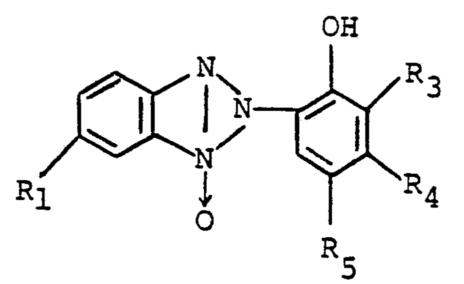

(II)

wherein $R_1$, $R_3$, $R_4$ and $R_5$ represent the same meanings as described above, in the presence of an alkali and

a naphthoquinone optionally in the presence of a surface active agent, characterized in that the reduction is carried out with one or more members selected from the group consisting of phosphorus, hypophosphorous acid and its salts.

2. A method as described in Claim 1, wherein the reaction temperature is in a range of 50°C to 200°C.

3. A method as described in Claim 2, wherein the reaction temperature is in a range of 60°C to 100°C.

4. A method as described in Claim 1, wherein the amount of the surface active agent is 0 to 10 wt.% based on the 2-phenylbenzotriazol-N-oxide.

5. A method as described in Claim 1, wherein the alkali is one or more members selected from the hydroxide, carbonate and hydrogencarbonate of alkali metals or alkaline earth metals.

6. A method as described in Claim 1, wherein the amount of the alkali is 2 to 6 moles based on 1 mole of the 2-phenylbenzotriazol-N-oxide.

7. A method as described in Claim 1, wherein the naphthoquinone is one or more members selected from 1,2-naphthoquinone, 1,4-naphthoquinone, 2,6-naphthoquinone, 2,3-dichloro-1,4-naphthoquinone and naphthoquinones substituted wth an alkyl group at the nucleus.

8. A method as described in Claim 1, wherein the amount of the naphthoquinone is 0.01 to 0.08 mole based on 1 mole of the 2-phenylbenzotriazol-N-oxide.

9. A method as described in Claim 1, wherein the amount of the phosphorus is 0.2 to 2 moles based on 1 mole of the 2-phenylbenzotriazol-N-oxide.

10. A method as described in Claim 1, wherein the amount of hypophosphorous acid or its salt is 0.5 to 4 moles based on 1 mole of 2-phenylbenzotriazol-N-oxide.

11. A method for producing a 2-phenylbenzotriazole represented by the general formula (III),

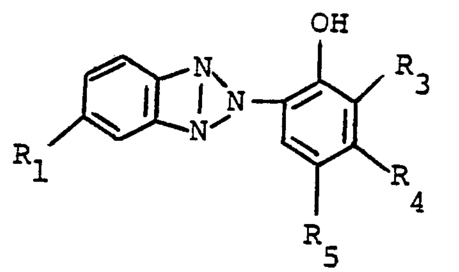

(III)

wherein $R_1$ represents a hydrogen or chlorine atom, $R_3$ and $R_5$ each represent a hydrogen atom, a $C_1$—$C_{12}$ alkyl group or a $C_1$—$C_8$ alkyl group substituted by phenyl group, and $R_4$ represents a hydrogen atom, a hydroxy group or a methoxy group, which comprises a) reducing an o-nitroazobenzene represented by the general formula (I),

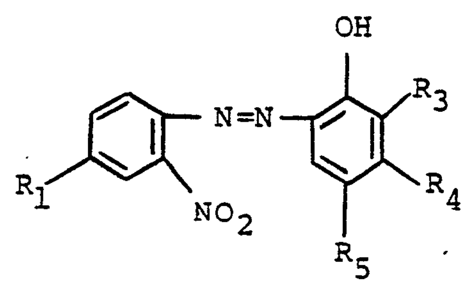

(I)

wherein $R_1$, $R_3$, $R_4$ and $R_5$ represent the same meanings as described above, to obtain a 2-phenylbenzotriazol-N-oxide represented by the general formula (II)

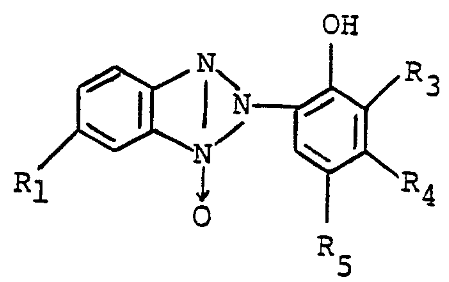

(II)

wherein $R_1$, $R_3$, $R_4$ and $R_5$ represent the same meanings as described above, and b) reducing the resulting

compound (II), both steps a) and b) being carrried out in the presence of an alkali and a naphthoquinone optionally in the presence of a surface active agent, characterized in that both reductions are carried out with one or more members selected from the group consisting of phosphorus, hypophosphorous acid and its salts.

12. A method as described in Claim 11, wherein the reaction temperature is in a range of 20°C to 50°C in the first step and is in a range of 60°C to 100°C in the second step.

13. A method as described in Claim 11, wherein the amount of the surface active agent is 0 to 10 wt.% based on the o-nitroazobenzene.

14. A method as described in Claim 11, wherein the alkali is one or more members selected from the hydroxide, carbonate and hydrogencarbonate of alkali metals or alkaline earth metals.

15. A method as described in Claim 11, wherein the amount of the alkali is 2 to 6 moles based on 1 mole of the o-nitroazobenzene.

16. A method as described in Claim 11, wherein the naphthoquinone is one or more members selected from 1,2-naphthoquinone, 1,4-naphthoquinone, 2,6-naphthoquinone, 2,3-dichloro-1,4-naphthoquinone and naphthoquinones substituted with an alkyl group at the nucleus.

17. A method as described in Claim 11, wherein the amount of the naphthoquinone is 0.002 to 0.08 mole based on 1 mole of the o-nitroazobenzene in the first step and is 0.01 to 0.1 mole based on 1 mole of the 2-phenylbenzotriazol-N-oxide in the second step.

18. A method as described in Claim 11, wheein the amount of the phosphorus is 0.2 to 2 moles based on 1 mole of the o-nitroazobenzene in the first step and is 0.2 to 2 moles based on 1 mole of the 2-phenylbenzotriazol-N-oxide in the second step.

19. A method as described in Claim 11, wherein the amount of the hypophosphorous acid or its salt is 0.5 to 4 moles based on 1 mole of the o-nitroazobenzene in the first step and is 0.5 to 4 moles based on 1 mole of the 2-phenylbenzotriazol-N-oxide.

**Patentansprüche**

1. Verfahren zur Herstellung eines 2-Phenylbenzotriazols der allgemeinen Formel (III)

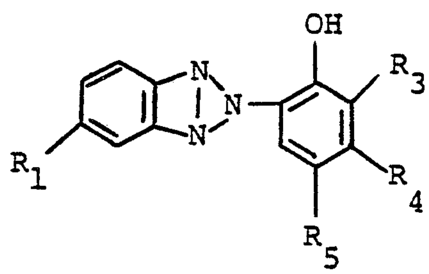

(III)

worin bedeuten:

$R_1$ ein Wasserstoff- oder Chloratom;

$R_3$ und $R_5$ jeweils ein Wasserstoffatom, eine $C_1$—$C_{12}$-Alkylgruppe oder eine phenylsubstituierte $C_1$—$C_8$-Alkylgruppe und

$R_4$ ein Wasserstoffatom, eine Hydroxygruppe oder eine Methoxygruppe,

durch Reduktion eines 2-Phenylbenzotriazol-N-oxids der allgemeinen Formel (II)

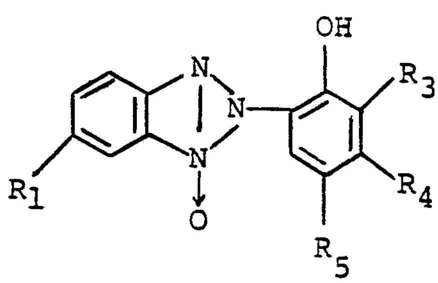

(II)

worin $R_1$, $R_3$, $R_4$ und $R_5$ die angegebene Bedeutung besitzten, in Gegenwart eines Alkalis und eines Naphthochinons sowie gegebenenfalls in Gegenwart eines oberflächenaktiven Mittels, dadurch gekennzeichnet, daß die Reduktion mit einem oder mehreren Mittel(n) aus der Gruppe Phosphor, Hypophosphorsäure und deren Salzen durchgeführt wird.

2. Verfahren nach Anspruch 1, dadurch gekennzeichnet, daß die Reaktionstemperatur im Bereich von 50°C bis 200°C liegt.

3. Verfahren nach Anspruch 2, dadurch gekennzeichnet, daß die Reaktionstemperatur im Bereich von 60°C bis 100°C liegt.

4. Verfahren nach Anspruch 2, dadurch gekennzeichnet, daß die Menge an oberflächenaktivem Mittel, bezogen auf das 2-Phenylbenzotriazol-N-oxid, 0—10 Gew.-% beträgt.

5. Verfahren nach Anspruch 1, dadurch gekennzeichnet, daß es sich bei dem Alkali um ein oder mehrere Mittel, ausgewählt aus Hydroxiden, Carbonaten und Hydrogencarbonaten von Alkalimetallen oder Erdalkalimetallen, handelt.

6. Verfahren nach Anspruch 1, dadurch gekennzeichnet, daß die Menge an dem Alkali, bezogen auf 1 Mol 2-Phenylbenzotriazol-N-oxid, 2—6 Mole beträgt.

7. Verfahren nach Anspruch 1, dadurch gekennzeichnet, daß es sich bei dem Naphthochinon um eines oder mehrere, ausgewählt aus 1,2-Naphthochinon, 1,4-Naphthochinon, 2,6-Naphthochinon, 2,3-Dichlor-1,4-naphthochinon und am Kern alkylsubstitiuerten Naphthochinonen, handelt.

8. Verfahren nach Anspruch 1, dadurch gekennzeichnet, daß die Menge an Naphthochinon, bezogen auf 1 Mol des 2-Phenylbenzotriazol-N-oxids, 0,01—0,08 Mol beträgt.

9. Verfahren nach Anspruch 1, dadurch gekennzeichnet, daß die Phosphormenge, bezogen auf 1 Mol 2-Phenylbenzotriazol-N-oxid, 0,2—2 Mole beträgt.

10. Verfahren nach Anspruch 1, dadurch gekennzeichnet, daß die Menge an Hypophosphorsäure oder einem Salz derselben, bezogen auf 1 Mol 2-Phenylbenzotriazol-N-oxid, 0,5—4 Mol(e) beträgt.

11. Verfahren zur Herstellung eines 2-Phenylbenzotriazols der allgemeinen Formel (III)

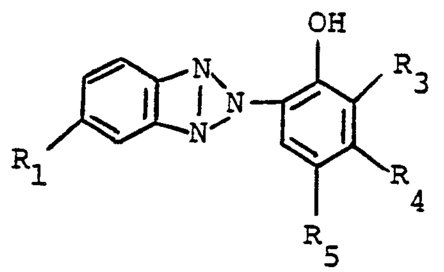

(III)

worin bedeuten:

$R_1$ ein Wasserstoff- oder Chloratom;

$R_3$ und $R_5$ jeweils ein Wasserstoffatom, eine $C_1$—$C_{12}$-Alkylgruppe oder eine phenylsubstituierte $C_1$—$C_8$-Alkylgruppe und

$R_4$ ein Wasserstoffatom, eine Hydroxygruppe oder eine Methoxygruppe,

durch

a) Reduktion eines o-Nitroazobenzols der allgemeinen Formel (I)

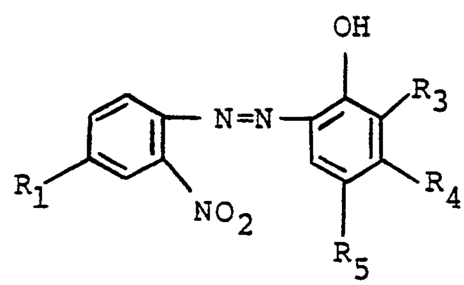

(I)

worin $R_1$, $R_3$, $R_4$ und $R_5$ die angegebene Bedeutung besitzen, zu einem 2-Phenylbenzotriazol-N-oxid der allgemeinen Formel (II)

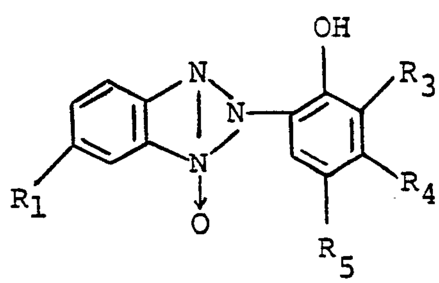

(II)

worin $R_1$, $R_3$, $R_4$ und $R_5$ die angegebene Bedeutung besitzen, und

b) Reduktion der erhaltenen Verbindung (II), wobei beide Stufen a) und b) in Gegenwart eines Alkalis und eines Naphthochinons und gegebenenfalls in Gegenwart eines oberflächenaktiven Mittels durchgeführt werden, dadurch gekennzeichnet, daß beide Reduktionen mit einem oder mehreren Mittel(n) aus der Gruppe Phosphor, Hypophosphorsäure und deren Salzen durchgeführt werden.

12. Verfahren nach Anspruch 11, dadurch gekennzeichnet, daß die Reaktionstemperatur in der ersten Stufe im Bereich von 20°C bis 50°C und in der zweiten Stufe im Bereich von 60°C bis 100°C liegt.

13. Verfahren nach Anspruch 11, dadurch gekennzeichnet, daß die Menge an oberflächenaktivem Mittel, bezogen auf das o-Nitroazobezol, 0—10 Gew.-% beträgt.

14. Verfahren nach Anspruch 11, dadurch gekennzeichnet, daß es sich bei dem Alkali um ein oder mehrere Mittel, ausgewählt aus den Hydroxiden, Carbonaten und Hydrogencarbonaten von Alkalimetallen oder Erdalkalimetallen, handelt.

15. Verfahren nach Anspruch 11, dadurch gekennzeichnet, daß die Menge an Alkali, bezogen auf 1 Mol o-Nitroazobenzol, 2—6 Mole beträgt.

16. Verfahren nach Anspruch 11, dadurch gekennzeichnet, daß es sich bei dem Naphthochinon um eines oder mehrere, ausgewählt aus 1,2-Naphthochinon, 1,4-Naphthochinon, 2,6-Naphthochinon, 2,3-Dichlor-1,4-naphthochinon und am Kern alkylsubstituierten Naphthochinonen, handelt.

17. Verfahren nach Anspruch 11, dadurch gekennzeichnet, daß die Menge an dem Naphthochinon in der ersten Stufe, bezogen auf 1 Mol des o-Nitroazobenzols, 0,002—0,08 Mol und in der zweiten Stufe, bezogen auf 1 Mol des 2-Phenylbenzotriazol-N-oxids, 0,01—0,1 Mol beträgt.

18. Verfahren nach Anspruch 11, dadurch gekennzeichnet, daß die Menge des Phospors in der ersten Stufe, bezogen auf 1 Mol des o-Nitroazobenzols, 0,2—2 Mole und in der zweiten Stufe, bezogen auf 1 Mol des 2-Phenylbenzotriazol-N-oxids, 0,2—2 Mole beträgt.

19. Verfahren nach Anspruch 11, dadurch gekennzeichnet, daß die Menge an der Hypophosphorsäure oder an deren Salz, in der ersten Stufe, bezogen auf 1 Mol des o-Nitroazobenzols, 0,5—4 Mol(e) und in der zweiten Stufe bezogen auf 1 Mol des 2-Phenylbenzotriazol-N-oxids, 0,5—4 Mol(e) beträgt.

**Revendications**

1. Procédé de préparation d'un 2-phénylbenzotriazole représenté par la formule générale (III)

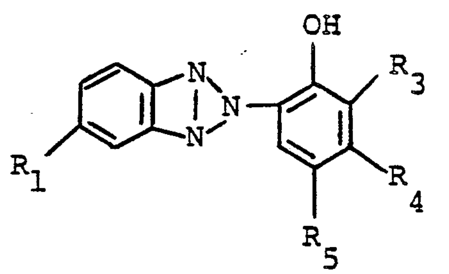

(III)

dans laquelle $R_1$ représente un atome d'hydrogène ou de chlore, $R_3$ et $R_5$ représentent chacun un atome d'hydrogène, un groupement alkyle en $C_1$—$C_{12}$ ou un groupement alkyle en $C_1$—$C_8$ substitué par un groupement phényle et $R_4$ représente un atome d'hydrogène ou un groupement hydroxy ou méthoxy, par réduction d'un N-oxyde de 2-phénylbenzotriazole représenté par la formule générale (II)

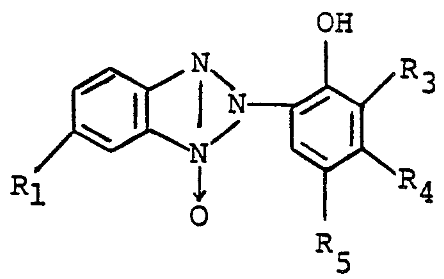

(II)

dans laquelle $R_1$, $R_3$, $R_4$ et $R_5$ ont les significations données précédemment, en présence d'une substance alcaline, d'une naphtoquinone et éventuellement en présence d'un surfactant, caractérisé en ce que la réduction est effectuée avec un ou plusieurs membres choisis dans le groupe comprenant le phosphore, l'acide hypophosphoreux et ses sels.

2. Procédé selon la revendication 1, dans lequel la température de réaction se situe dans la gamme de 50°C à 200°C.

3. Procédé selon la revendication 2, dans lequel la température de réaction se situe dans la gamme de 60°C à 100°C.

4. Procédé selon la revendication 1, dans lequel la quantité de surfactant est de 0 à 10% en poids sur la base du N-oxyde de 2-phénylbenzotriazole.

5. Procédé selon la revendication 1, dans lequel la substance alcaline est constituée par un ou plusieurs membres choisis parmi les hydroxydes, carbonates et hydrogènocarbonates de métaux alcalins ou de métaux alcalino-terreux.

6. Procédé selon la revendication 1, dans lequel la quantité de substance alcaline est de 2 à 6 moles pour 1 mole du N-oxyde de 2-phénylbenzotriazole.

7. Procédé selon la revendication 1, dans lequel la naphtoquinone est constituée par un ou plusieurs membres choisis parmi la 1,2-naphtoquinone, la 1,4-naphtoquinone, la 2,6-naphtoquinone, la 2,3-dichloro-1,4-naphtoquinone et les naphtoquinones substituées par un groupement alkyle sur le noyau.

8. Procédé selon la revendication 1, dans lequel la quantité de naphtoquinone est de 0,01 à 0,08 mole pour 1 mole du N-oxyde de 2-phénylbenzotriazole.

9. Procédé selon la revendication 1, dans lequel la quantité de phosphore est de 0,2 à 2 moles pour 1 mole du N-oxyde de 2-phènylbenzotriazole.

10. Procédé selon la revendication 1, dans lequel la quantité d'acide hypophosphoreux ou de son sel est de 0,5 à 4 moles pour 1 mole de N-oxyde de 2-phénylbenzotriazole.

11. Procédé de préparation d'une 2-phénylbenzotriazole représenté par la formule générale (III)

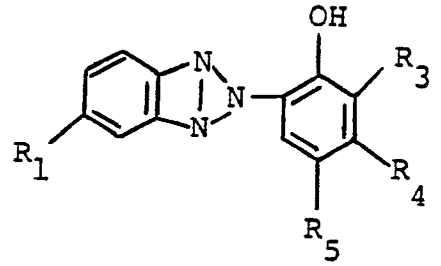

(III).

dans laquelle $R_1$ représente un atome d'hydrogène ou de chlore, $R_3$ et $R_5$ représentent chacun un atome d'hydrogène, un groupement alkyle en $C_1$—$C_{12}$ ou un groupement alkyle en $C_1$—$C_8$ substitué par un groupement phényle et $R_4$ représente un atome d'hydrogène ou un groupement hydroxy ou méthoxy, comprenant les opérations consistant (a) à réduire un o-nitroazobenzène représenté par la formule générale (I)

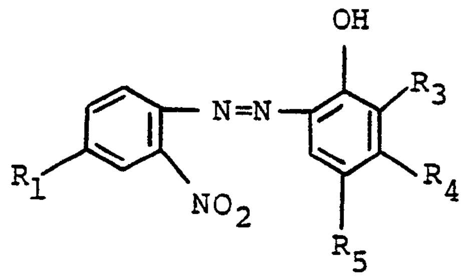

(I)

dans laquelle $R_1$, $R_3$, $R_4$ et $R_5$ ont les significations données précédemment, pour obtenir un N-oxyde de 2-phénylbenzotriazole représenté par la formule générale (II)

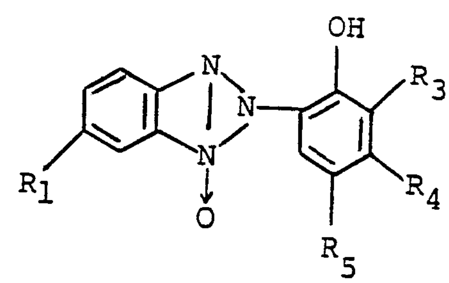

(II)

dans laquelle $R_1$, $R_3$, $R_4$ et $R_5$ ont les significations données précédemment, et (b) à réduire le composé (II) résultant, les deux étapes (a) et (b) étant conduites en présence d'une substance alcaline, d'une naphtoquinone et éventuellement en présence d'un surfactant, caractérisé en ce que les deux réductions sont effectuées avec un ou plusieurs membres choisis dans le groupe comprenant le phosphore, l'acide hypophosphoreux et ses sels.

12. Procédé selon la revendication 11, dans lequel la température de réaction se situe dans la gamme de 20°C à 50°C dans la première étape et dans la gamme de 60°C à 100°C dans la seconde étape.

13. Procédé selon la revendication 11, dans lequel la quantité de surfactant est de 0 à 10% en poids sur la base de l'o-nitroazobenzène.

14. Procédé selon la revendication 11, dans lequel la substance alcaline est constituée par un ou plusieurs membres choisis parmi les hydroxydes, carbonates et hydrogénocarbonates de métaux alcalins ou de métaux alcalino-terreux.

15. Procédé selon la revendication 11, dans lequel la quantité de substance alcaline est de 2 à 6 moles pour 1 mole de l'o-nitroazobenzène.

16. Procédé selon la revendication 11, dans lequel la naphtoquinone est constituée par un ou plusieurs membres choisis parmi la 1,2-naphtoquinone, la 1,4-naphtoquinone, la 2,6-naphtoquinone, la 2,3-dichloro-1,4-naphtoquinone et les naphtoquinones substituées par un groupement alkyle sur le noyau.

17. Procédé selon la revendication 11, dans lequel la quantité de naphtoquinone est de 0,002 à 0,08 mole pour 1 mole de l'o-nitroazobenzène dans la première étape et de 0,01 à 0,1 mole pour 1 mole du N-oxyde de 2-phénylbenzotriazole dans la seconde étape.

18. Procédé selon la revendication 11, dans lequel la quantité de phosphore est de 0,2 à 2 moles pour 1 mole de l'o-nitroazobenzène dans la première étape et de 0,2 à 2 moles pour 1 mole du N-oxyde de 2-phénylbenzotriazole dans la seconde étape.

19. Procédé selon la revendication 11, dans lequel la quantité d'acide hypophosphoreux ou de son sel est de 0,5 à 4 moles pour 1 mole de l'o-nitroazobenzène dans la première étape et de 0,5 à 4 moles pour 1 mole du N-oxyde de 2-phénylbenzotriazole dans la seconde étape.